# EUROPEAN PATENT SPECIFICATION

(11) **EP 2 720 742 B1**
(45) Date of publication and mention of the grant of the patent: **17.02.2021**
(21) Application number: 12799978.7
(22) Date of filing: 12.06.2012
(51) Int. Cl.: A61M 11/00, A61K 9/00, C08F 6/00

(54) **MEDICINAL INHALATION DEVICES, VALVES AND COMPONENTS THEREOF**
MEDIZINISCHE INHALATIONSVORRICHTUNGEN SOWIE VENTILE DAFÜR UND KOMPONENTEN DAVON
DISPOSITIFS D'INHALATION DE MÉDICAMENT, SOUPAPES ET COMPOSANTS ASSOCIÉS

(30) Priority: 15.06.2011 GB 201110058
(43) Date of publication of application: 23.04.2014
(73) Proprietor: Kindeva Drug Delivery L.P., St. Paul, MN 55170 (US)
(72) Inventor: JINKS, Philip, A., Bracknell Berkshire RG12 8HT (GB)
(74) Representative: Isarpatent
(86) International application number: PCT/US2012/042023
(87) International publication number: WO 2012/173971

(56) References cited:
- EP-A1- 2 275 479
- US-A1- 2003 148 030
- US-A1- 2005 107 870
- US-A1- 2007 062 979
- US-A1- 2008 066 739
- US-A1- 2010 015 238
- US-A1- 2010 199 983
- Chuispastonbot: "Stearic acid - Wikipedia, the free encyclopedia", , 13 May 2011 (2011-05-13), XP055178134, Retrieved from the Internet: URL:http://en.wikipedia.org/w/index.php?ti tle=Stearic_acid&oldid=428937211#cite_note -5 [retrieved on 2015-03-20]
- 24.223.197.194: "Magnesium stearate - Wikipedia, the free encyclopedia", , 12 February 2011 (2011-02-12), XP055178135, Retrieved from the Internet: URL:http://en.wikipedia.org/w/index.php?ti tle=Magnesium_stearate&oldid=413414785 [retrieved on 2015-03-20]

## Description

### Field of the Invention

The present invention relates to medicinal inhalation devices, metered dose valves and valve components for such devices as well as methods of making such metered dose valves and components thereof.

### Background of the Invention

Medicinal inhalation devices, in particular pressurized inhalers, such as pressurized metered dose inhalers (pMDIs), are widely used for delivering medicaments.

Pressurized metered dose medicinal inhalation devices typically comprise a plurality of hardware components. In the case of pMDIs, these include metered dose valves including their individual components, such as ferrules, valve bodies, valve stems, tanks, springs retaining cups and seals. Metered dose valves and their components have a number of surfaces, some of which contact or may come into contact with a surface of another component.

Often a needed and/or desired material for a particular component is found to be unsuitable in regard to its surface properties, e.g. surface energy. The use of materials having relatively high surface energy for certain components, e.g. metered dose valves and/or individual components thereof, may have undesirable effects for the operation of movable components of the valve and hence operation of the medicinal inhalation device. For example, the seals in metered dose valves generally have high surface energies due to their rubbery natural. Another example is the relatively high surface energy of acetal polymer for valve stems. Such high surface energies of seals and/or valve stems can bring about a high friction between the valve stem and the seal(s) as they pass along one another during actuation. Such high friction typically impacts the force to fire and the force of return of the valve, which in turn generally has a consequent impact on the uniformity of valve actuation, which then in turn may in some cases impact the uniformity of the medicinal delivery. Other examples of potentially undesirable effects as a result of high friction of surfaces of components passing one another may include undesirable wear of the surfaces and/or an increase in the friction over the lifetime of the device, which may lead to, in a worse case, sticking or even seizing of the valve.

In the past it was common to add surfactants soluble in the liquefied propellant to the aerosol formulation. However the customary surfactants used in CFC-containing PMDIs, i.e. oleic acid, sorbitan trioleate, and lecithin are only partially soluble in the hydrofluoroalkanes, 1,1,1,2-tetrafluoroethane (HFA 134a) and 1,1,1,2,3,3,3-heptafluoropropane (HFA 227), used to replace CFC as propellants in metered dose aerosols. Various other types of excipients have been proposed for use with HFA 134a and/or HFA 227 containing PMDIs, see e.g. EP 0536235, EP 0504 112, EP0605578, US 5,415,853, WO94/21228-9, US 5,492,688, EP 0633019, US 5,502,076, US 5,508,023, and US 2004/101483. However most are unsuccessful and/or undesirable. In regard to the latter, the use of formulation excipients have become generally undesirable. This is related to the fact that regulatory agencies have become more stringent e.g. in regard to toxicology testing and the extent of such testing of such excipients.

Various coatings have been proposed for particular components or surfaces of metered dose inhalers, see e.g. EP 642 992, WO 96/32099, WO 96/32150-1, WO 96/32345, WO 99/42154, WO 02/47829, WO03/024623, WO 02/30498, WO 01/64273, WO 91/64274-5, WO 01/64524, and WO 03/006181. However quite a number are related to coating the aerosol cans, and some valve components that are critical in terms of friction, e.g. seals, normally can not be coated by such methods or coatings.

US 2003/0148030A1 discloses polymeric coatings, method for coating articles and such articles.

### BACKGROUND OF THE INVENTION

In some cases, valve components or valve sub-assemblies (for example a valve "core" made up of the valve stem, seal(s), and spring) are siliconized (e.g. tumbling in silicone oil). While this may lower friction between e.g. the valve stem and the seal(s), it has been observed that siliconization may also increase the propensity of medicament deposition on the respective parts.

### Summary of the Invention

Although a number of approaches have been proposed, there is an ongoing need for metered dose valves and/or components thereof for medicinal inhalation devices (in particular pMDIs) having desirable low friction as well as convenient methods of providing such metered dose valves and components.

In one aspect of the present invention there is provided a method of making a metered dose valve for use in a medicinal inhalation device or a component of a metered dose valve for in use a medicinal inhalation device, wherein at least a portion of a surface of the valve or component, respectively, is to be coated, the method comprising the steps: a) providing the valve or the component, respectively; and b) forming a coating on said at least a portion of a surface of the valve or the component, respectively, wherein said coating comprises a particulate material selected from the group consisting of Magnesium Stearate, Calcium Stearate, Zinc Stearate, Aluminium Stearate, Stearic acid, Magnesium Palmitate, Calcium Palmitate, Zinc Palmitate or Aluminium Palmitate, Palmitic acid, and mixtures thereof, characterized in that the step of forming a coating on said at least a portion of a surface of the valve (10) or the component, respectively, comprises
contacting said at least a portion of a surface of the valve (10) or the component, as applicable, with particulate material in dry form to provide said coating, wherein the particulate material is de-agglomerated, such that at least 90 % by weight of the particulate material passes or would pass, as applicable, a 325 mesh; or
contacting said at least a portion of a surface of the valve (10) or the component, as applicable, with particulate material suspended in a fluid and then removing the fluid to provide said coating, wherein the fluid comprises a volatile hydrocarbon, a volatile alcohol, a volatile ester, a volatile fluorocarbon or a mixture thereof..

In another aspect of the present invention the above method of making a metered dose valve for use in a medicinal inhalation device, wherein at least a portion of a surface of a component of the valve is to be coated, comprises the steps: a) providing the component of the valve; b) forming a coating on said at least a portion of a surface of the component; and c) assembling the valve using said coated component and, as applicable, other valve-components.

Favorably, singular parts (e.g. seals or valve stems) or subassemblies are coated. Advantageously, the component is a component that comes into contact with a movable component or is movable during storage or delivery from the medicinal inhalation device.

The coating of seals or components comprising a seal (e.g. subassemblies comprising seals) or components coming into contact with a seal have been found particularly advantageous. When assembling a valve, one component part (e.g. just the seal) may have been treated or a number of component parts may have be treated (e.g. just the seal and valve stem) or all the component parts may have been treated.

Surprisingly the coating with the aforesaid particulate material, prior to attachment of the valve to the medicinal container (e.g. aerosol can) of the metered dose inhalation device, provides a favorably durable, low friction surface for desirable valve function.

Without wishing to be bound by theory, it appears for example that during dry coating of seals that small particulates of the selected coating material (e.g. commercial Magnesium Stearate), smear out across elastomeric surfaces and become in part embedded in the surface so that an adherent, low friction coating is presented that is not easily washed off (nor dissolved off due to low solubility) in HFA 134a and/or HFA 227 propellant systems. Accordingly the step of forming the coating on said at least a portion of a surface of the valve or the component, respectively, comprise contacting said at least a portion of a surface of the valve or the component, as applicable, with said particulate material in dry form to provide said coating. For example, the mixing, tumbling or shaking of seals in dry particulate material has been found to provide generally uniform coating on the exposed surfaces, advantageously both on the bores as well as the faces. Dry coating is a convenient and desirable method of coating seals.

Alternatively the step of forming comprise contacting said at least a portion of a surface of the valve or the component, as applicable, with particulate material suspended in a fluid and then removing the fluid to provide particulate material coating (e.g. dipping or spray coating). It has been found that evaporation of a suspension of particulate material (such as sub-micron Magnesium Stearate) from components generally provides an even distribution of powder coated onto the surface facilitated by surface tension in the transient meniscus provided by the selected suspending fluid (e.g. ethanol) which pulls the particles towards the surface of the elastomeric or other material onto which it is coated. Wet coating is a convenient and desirable method of coatings valve stems, springs and subassemblies, such as cores, i.e. subassemblies of the valve stem and seal(s) and, if applicable a spring.

For the provision of favorable lubricating properties, desirably the particulates of the particulate material are flat or plate-like. The particulates of the particulate material desirably have a mass mean aspect ratio (ratio of a particulate's longest dimension to its shortest dimension) equal to or greater than 5, more desirably equal to or greater than 10. Aspect ratio may be determined for example by techniques known in the art such as microscopy with image analysis, or combined use of aerodynamic particle sizing or surface area measurement with microscopy or laser diffraction.

Also in regard to facilitating lubrication and desirable coat formation, it has been found desirable that the particulates of the particulate materials are substantially completely de-agglomerated and/or have low average particle size and/or have higher surface area. Moreover, favorably the particulate material is de-agglomerated such that at least 90 % by weight of the particulate material passes or would pass, as applicable, a 325 mesh, more favorably at least 90 % by weight of the particulate material passes or would pass, as applicable, a 400 mesh, most favorably at least 95 % by weight of that particulate material passes or would pass, a applicable, a 400 mesh. The mass median diameter of the particulates of the particulate material, e.g. as determined by Malvern Laser Diffraction, are favorably generally at most 30 microns, more favorably at most 25 microns, even more favorably at most 20 microns, and most favorably at most 15 microns. Desirably the specific surface area (e.g. a BET specific surface area) of the particulate material is equal to or greater than 2 m²/g, in particular equal to or greater than 3 m²/g.

Further described are:
A metered dose valve of a medicinal inhalation device, wherein at least a portion of a surface of a component of the valve is coated prior to attachment of the valve to a medicinal container of the medicinal inhalation device and wherein said coating comprises a particulate material selected from the group consisting of Magnesium Stearate, Calcium Stearate, Zinc Stearate, Aluminium Stearate, Stearic acid, Magnesium Palmitate, Calcium Palmitate, Zinc Palmitate or Aluminium Palmitate, Palmitic acid, and mixtures thereof.
A medicinal inhalation device comprising a metered dose valve, wherein at least a portion of a surface of a component of the valve is coated, said coating comprising a particulate material selected from the group consisting of Magnesium Stearate, Calcium Stearate, Zinc Stearate, Aluminium Stearate, Stearic acid, Magnesium Palmitate, Calcium Palmitate, Zinc Palmitate or Aluminium Palmitate, Palmitic acid, and mixtures thereof, and wherein the medicinal aerosol formulation filled into the device is essentially free of Magnesium Stearate, Calcium Stearate, Zinc Stearate, Aluminium Stearate, Stearic acid, Magnesium Palmitate, Calcium Palmitate, Zinc Palmitate or Aluminium Palmitate, and Palmitic acid.

Metered dose inhalation devices described herein, in particular pMDIs, are advantageous in that favorable valve performance may be achieved, from the first shot, without having Magnesium Stearate, Calcium Stearate, Zinc Stearate, Aluminium Stearate, Stearic acid, Magnesium Palmitate, Calcium Palmitate, Zinc Palmitate or Aluminium Palmitate, Palmitic acid, and mixtures thereof as a formulation excipient (i.e. essentially free of said materials). This is desirable for the patient in that administration of non-medicinal materials may be minimized and for the pharmaceutical company in that design and formulation work may be simplified.

The above summary of the present invention is not intended to describe each disclosed embodiment or every implementation of the present invention. The description that follows more particularly exemplifies illustrative embodiments. Also further embodiments are described in dependent claims. In several places throughout the application, guidance is provided through lists of examples, which examples can be used individually and in various combinations. In each instance, the recited list serves only as a representative group and should not be interpreted as an exclusive list

### Brief Description of Drawings

The invention will now be described with reference to the accompanying drawings in which:
Figure 1a represents a schematic cross-sectional view of a pressurized metered dose inhaler known in the art and Figure 1b represents an enlarged view of a portion of the inhaler.
Figures 2 to 5 represent schematic cross-sectional views of further metered dose valves known in the art for use in pressurized metered dose inhalers.
Figures 6a to 6c represent force to fire, return force and friction results of a through life testing of pMDIs fitted with valves with components untreated, siliconized and wet coated.
Figure 7 is a photograph of untreated and a series of dry coated metered dose valve seals.
Figure 8 is a photograph of an apparatus used to measure Angle of Slip.
Figure 9 is a box plot of results of Angle of Slip measurements of untreated and a number of dry coated metered dose valve seals.

### Detailed Description

It is to be understood that the present invention covers all combinations of particular, suitable, desirable, favorable, advantageous and preferred aspects of the invention described herein.

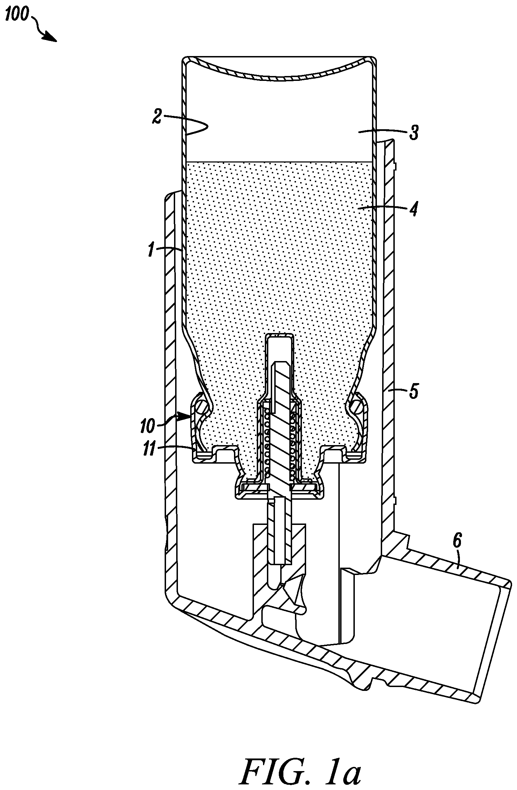

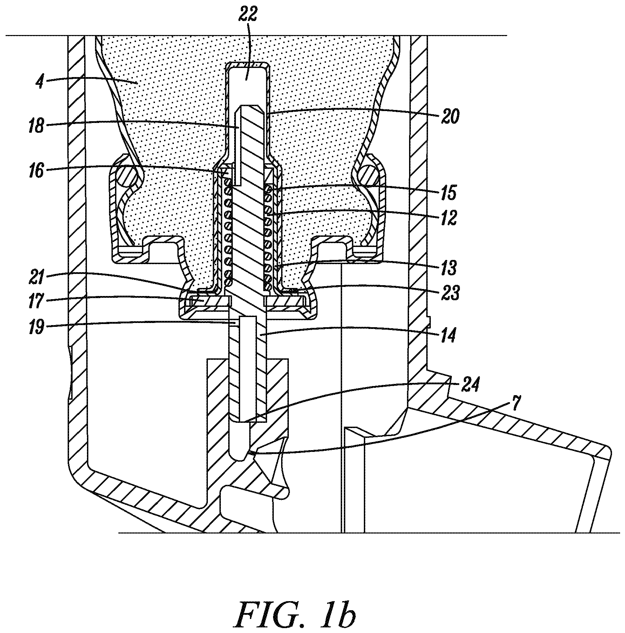

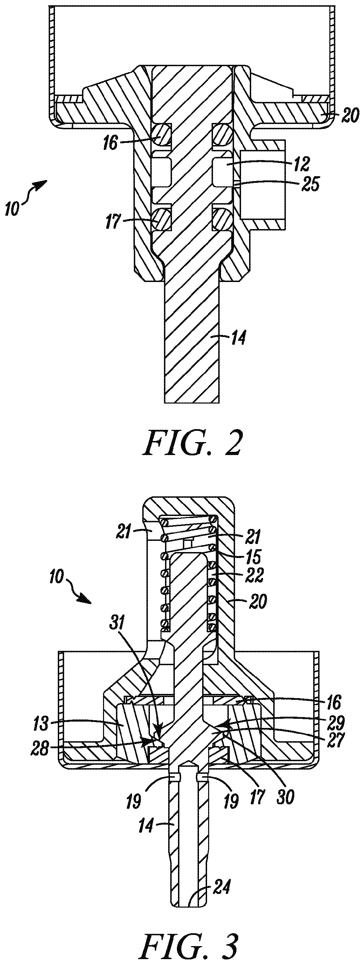

For better understanding of the present invention, in the following an exemplary, well known pressurized metered dose inhaler (Figure 1) as well as several known metered dose valves for pressurized metered dose inhalers (Figures 2 to 5) will be first described. In particular, Figure 1a shows a metered dose dispenser (100), in particular an inhaler, including an aerosol container (1) fitted with a metered dose valve (10) (shown in its resting position).

Aerosol containers for metered dose inhalers are typically made of aluminum or an aluminum alloy. Aerosol containers may be made of other materials, such as stainless steel, glass, plastic or ceramics.

Returning to Figure 1a, the valve is typically affixed onto the container via a cap or ferrule (11) (typically made of aluminum or an aluminum alloy) which is generally provided as part of the valve assembly. The illustrated valve is a commercial valve marketed under the trade designation SPRAYMISER by 3M Company, St. Paul, Minnesota, USA. As shown in Figure 1a, the container/valve dispenser is typically provided with an actuator (5) including an appropriate patient port (6), such as a mouthpiece. For administration to the nasal cavities the patient port is generally provided in an appropriate form (e.g. smaller diameter tube, often sloping upwardly) for delivery through the nose. Actuators are generally made of a plastic, for example polypropylene or polyethylene. As can be seen from Figure 1a, the inner walls (2) of the container and the outer walls of the portion(s) of the metered dose valve located within the container defined a formulation chamber (3) in which aerosol formulation (4) is contained. Depending on the particular metered dose valve and/or filling system, aerosol formulation may be filled into the container either by cold-filling (in which chilled formulation is filled into the container and subsequently the metered dose valve is fitted onto the container) or by pressure filling (in which the metered dose valve is fitted onto the container and then formulation is pressure filled through the valve into the container).

An aerosol formulation used in a metered dose inhaler typically comprises a medicament or a combination of medicaments and liquefied propellant selected from the group consisting of HFA 134a, HFA 227 and mixtures thereof.

Medicament may be provided in particulate form (generally having a mass median size in the range of 1 to 10 microns) suspended in the liquefied propellant. Alternatively medicament may be in solution (e.g. dissolved) in the formulation. In the event a combination of two or more medicaments is included, all the medicaments may be suspended or in solution or alternatively one or more medicaments may be suspended, while one or more medicaments may be in solution. A medicament may be a drug, vaccine, DNA fragment, hormone or other treatment. The amount of medicament would be determined by the required dose per puff and available valve sizes, which are typically 25, 50 or 63 microlitres, but may include 100 microlitres where particularly large doses are required. Suitable drugs include those for the treatment of respiratory disorders, e.g., bronchodilators, anti-inflammatories (e.g. corticosteroids), anti-allergics, anti-asthmatics, anti-histamines, and anti-cholinergic agents. Therapeutic proteins and peptides and monoclonal antibodies may also be employed for delivery by inhalation. Exemplary drugs which may be employed for delivery by inhalation include but are not limited to: albuterol, terbutaline, ipratropium, oxitropium, tiotropium, aclidinium, glycopyrronium, beclomethasone, flunisolide, budesonide, mometasone, ciclesonide, cromolyn sodium, nedocromil sodium, ketotifen, azelastine, ergotamine, cyclosporine, salmeterol, fluticasone, formoterol, procaterol, indacaterol, TA2005, vilanterol, omalizumab, zileuton, insulin, pentamidine, calcitonin, leuprolide, alpha-1-antitrypsin, interferons, triamcinolone, and pharmaceutically acceptable salts and esters thereof such as albuterol sulfate, formoterol fumarate, salmeterol xinafoate, vilanterol terfenetate, beclomethasone dipropionate, triamcinolone acetonide, fluticasone propionate, fluticasone furoate, tiotropium bromide, aclidininium bromide, glycopyrronium bromide, leuprolide acetate and mometasone furoate.

Described herein are metered dose valves or medicinal inhalation devices with metered dose valves, comprising a coating on at least a portion of a surface of a component of the valve. The coating comprises a particulate material selected from the group consisting of Magnesium Stearate, Calcium Stearate, Zinc Stearate, Aluminium Stearate, Stearic acid, Magnesium Palmitate, Calcium Palmitate, Zinc Palmitate or Aluminium Palmitate, Palmitic acid, and mixtures thereof. Advantageously, the coating is on the component(s) prior to fitting the valve to a medicinal container (e.g. aerosol can) of the medicinal inhalation device.

As mentioned above, medicinal inhalation devices described herein, in particular pMDIs, are advantageous in that favorable valve performance may be achieved , from the first shot, whereby the formulation originally filled in to the device is essentially free (less than 0.0001 wt % with respect to the formulation) or more particularly free, of Magnesium Stearate, Calcium Stearate, Zinc Stearate, Aluminium Stearate, Stearic acid, Magnesium Palmitate, Calcium Palmitate, Zinc Palmitate or Aluminium Palmitate, and Palmitic acid.

Metered dose valves or medicinal inhalation devices with metered dose valves described herein are particularly useful in that the medicinal aerosol formulation may be substantially free of solubilized surfactant (0.005 wt % with respect to the formulation); or is essentially free (less than 0.0001 wt % with respect to the formulation) or free of a solubilized surfactant. Alternatively or additionally, metered dose valves or medicinal inhalation devices with metered dose valves described in detail below, are particularly useful in metered dose inhalers including a medicinal aerosol formulation that contains low amounts of ethanol (less than 5 wt % with respect to the formulation), or is substantially free (less than 0.1 wt % with respect to the formulation) or free of ethanol. Alternatively, metered dose valves or medicinal inhalation devices with metered dose valves described herein may include a medicinal aerosol formulation that contains relatively high amounts of ethanol e.g. 5 to 15 wt %.

Embodiments in accordance with the invention may, as desired or needed, comprise other formulation excipients, such as glycerol, ascorbic acid, mineral acid such as hydrochloric acid, CO₂, N₂O or a particulate bulking agent.

The valve shown in Figure 1a, better viewed in Figure 1b, includes a metering chamber (12), defined in part by an inner valve body (13), through which a valve stem (14) passes. The valve stem, which is biased outwardly by a compression spring (15), is in sliding sealing engagement with an inner tank seal (16) and an outer diaphragm seal (17). The valve also includes a second valve body (20) in the form of a bottle emptier.

(For the sake of clarity in the description of various metered dose valves, in particular those including at least two valve bodies, in the following a valve body defining in part the metering chamber will be referred to as a "primary" valve body, while other types of valve body, e.g. defining a pre-metering region, a pre-metering chamber, a spring cage and/or a bottle emptier will be referred to as a "secondary" valve body.)

Returning to Figure 1a, aerosol formulation (4) can pass from the formulation chamber into a pre-metering chamber (22) provided between the secondary valve body (20) and the primary valve body (13) through an annular space (21) between the flange (23) of the secondary valve body and the primary valve body. To actuate (fire) the valve, the valve stem (14) is pushed inwardly relative to the container from its resting position shown in Figures 1a and b, allowing formulation to pass from the metering chamber through a side hole (19) in the valve stem and through a stem outlet (24) to an actuator nozzle (7) then out to the patient. When the valve stem (14) is released, formulation enters into the valve, in particular into the pre-metering chamber (22), through the annular space (21) and thence from the pre-metering chamber through a groove (18) in the valve stem past the tank seal (16) into the metering chamber (12).

As mentioned above, Figures 2 to 5 show other known metered dose valves used in pMDIs. Similar to the valve shown in Figure 1, the valves of Figures 2 to 5 are typically fitted via a ferrule onto an aerosol container whereby a formulation chamber is defined by the inner walls of the container and the outer walls of the portion(s) of the valve located within the container. For the sake of ease in understanding and comparison, similar components of the respective valves are identified with like reference numbers in the Figures.

Figure 2 shows a metered dose valve (10) of a type generally similar to that disclosed and described in US Patent 5,772,085. The valve is shown in its resting position and includes a valve body (20) and a valve stem (14). The valve stem, which is biased outwardly under the pressure of the aerosol formulation contained within the formulation container, is provided with an inner seal and an outer seal (16 and 17). Unlike the valves in Figure 1 and Figures 3 to 5, which are push-to-fire type valves, the valve here is a release-to-fire type valve. To actuate the valve, the valve stem (14) is first pushed upwards into the formulation chamber (not shown), so that the outer seal (17) passes inwardly beyond an outlet (25) provided in the external portion of the valve body and the inner seal (16) then passes inwardly and disengages from the inner walls of the valve body, thus bringing the metering chamber (12) up into the formulation chamber so that formulation can enter the metering chamber (referred to as the priming position of the valve) and then the valve stem is released moving outwardly so that the inner seal re-engages the valve body and the outer seal then passes outwardly beyond the outlet, bringing the metering chamber in communication with the outlet, so that formulation passes through the outlet to the patient.

Figure 3 shows a metered dose valve (10) of the type generally similar to that disclosed and described in WO 2004/022142. The valve is shown in its resting position and includes a secondary valve body (20) and a valve stem (14) that is biased outwardly by a compression spring (15). The valve is provided with an inner seal (16) and outer diaphragm seal (17), with the valve stem being in sliding sealing engagement with the diaphragm seal. In this valve, the secondary valve body is in the form of a spring cage housing having three slots (21, two visible) providing communication between the formulation chamber (not shown) and a pre-metering chamber (22). This valve includes a transitory metering chamber formed upon actuation of the valve. During actuation of the valve, as the valve stem (14) is pushed inwardly relative to the container, a metering chamber (12, not visible) is formed between a lower surface (28) of a conical portion (27) of the valve stem (14) and an upper, sloping surface (31) of a primary valve body (13). Aerosol formulation passes around the shoulder (30) of the conical portion of the valve stem into the forming metering chamber and as the valve stem is further pushed in the upper surface (29) of the conical portion forms a face seal with the inner seal (16), thereby sealing off the metering chamber. As the valve stem is yet further displaced inwardly, formulation is allowed to pass from the metering chamber through side holes (19) in the valve stem and through a stem outlet (24) in the valve stem, and subsequently out to the patient typically via an actuator nozzle (7, not shown).

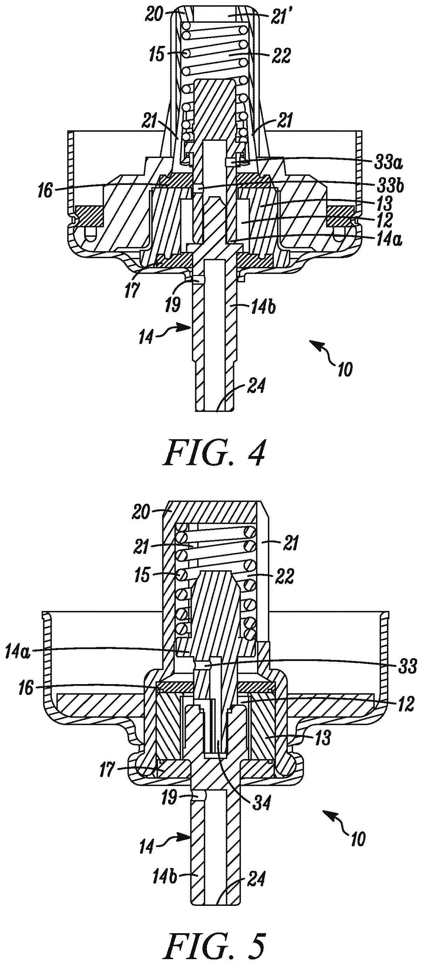

Figure 4 shows a commercial metered dose valve supplied by Bespak, Bergen Way, King's Lynn, Norfolk, PE30 2JJ, UK under the trade designation BK357, in its resting position. The valve includes a secondary valve body (20) in the form of a spring cage with two slots (21) and an opening at the top (21') allowing communication between the formulation chamber (not shown) and a pre-metering chamber (22). The valve also includes a valve stem (14), made of two components (14a, 14b), which is biased outwardly by a compression spring (15) and passes through a metering chamber (12) defined in part by a primary valve body (13). The valve stem is in sliding sealing engagement with an inner seal (16) and an outer diaphragm seal (17). Aerosol formulation can pass from the pre-metering chamber (22) into the metering chamber (12) via side holes (33a, 33b) in the upper portion (14a) of the stem (14). Similar to the valve shown in Figure 1, to actuate (fire) the valve, the valve stem (14) is pushed inwardly relative to the container, allowing a metered dose of formulation to pass from the metering chamber through a side hole (19) in the valve stem and through a stem outlet (24) and then typically through an actuator nozzle (7, not shown) out to the patient.

Figure 5 shows a commercial metered dose valve supplied by Valois SAS, Pharmaceutical Division, Route des Falaises, 27100 le Vaudreuil, France under the trade designation RCS, in its resting position. The valve includes a secondary valve body (20) in the form of a spring cage with three slots (21, two visible) allowing communication between the formulation chamber (not shown) and a pre-metering chamber (22). The valve also include a valve stem (14), made of two components (14a, 14b), which is biased outwardly by a compression spring (15) and passes through a metering chamber (12) defined in part by a primary valve body (13). The valve stem is in sliding sealing engagement with an inner seal (16) and an outer diaphragm seal (17). Aerosol formulation can pass from the pre-metering chamber (22) into the metering chamber through a side hole (33) and an internal channel (34) provided in the upper portion (14a) of the valve stem. Similar to the valve shown in Figure 1, to actuate (fire) the valve, the valve stem (14) is pushed inwardly relative to the container, allowing formulation to pass from the metering chamber through a side hole (19) in the valve stem and through a stem outlet (24) and then typically through an actuator nozzle (7, not shown) out to the patient.

With the exception of the elastomeric seals used in metered dose valves, typically the components of such valves are made of metal (e.g. stainless steel, aluminum or aluminum alloy) or plastic. For example compression springs are generally made of a metal, in particular stainless steel as the conventional material. Compression springs may also be made of aluminum or aluminum alloy. Valve stems and valve bodies are generally made of metal and/or plastic; as a metal conventionally stainless steel is used (other metals that may be used include aluminum, aluminum alloy and titanium) and as plastics conventionally polybutylene terephthalate (PBT) and/or acetal are used (other polymers that may be used include polyetheretherketones (PEEK), polymethylpentene, polyphenylene sulphide, thermotropic liquid crystalline polymer, PTFE or nylon, other polyesters (such as tetrabutylene terephthalate), polycarbonates and polyethylene).

Seals are typically elastomeric. Seals are generally made of polybutadiene-acrylonitrile (Nitrile) polymer, polychloroprene (Neoprene), polyethylene-propylene-diene-modified (EPDM), polyisobutylene-isoprene (Butyl), or chlorinated polyisobutylene-isoprene (Chlorobutyl), each compounded with suitable fillers cross-linking agents and processing aids. Seals may also be compounded from thermoplastic elastomeric materials, e.g. Flexomer ® DFDB1085(ex Dow), Santoprene ® (ex Advanced Elastomer Systems), or mixtures of thermosetting elastomers with thermoplastic materials.

Embodiments in accordance with other aspects of the present invention include forming a coating on at least a portion of a surface of a metered dose valve or a component thereof, where the coating comprises a particulate material selected from the group consisting of Magnesium Stearate, Calcium Stearate, Zinc Stearate, Aluminium Stearate, Stearic acid, Magnesium Palmitate, Calcium Palmitate, Zinc Palmitate or Aluminium Palmitate, Palmitic acid, and mixtures thereof. Suitable stearates, stearic acids, palmitates, palmitic acids for use are commercially available. Typically commercial stearates include in part palmitates.

Desirably a component treated in accordance with aspects of the present invention described herein is a component of a metered dose valve used in a metered dose inhaler, in particular in a pMDI. Favorably at least a portion of a surface, more favorably the entire surface, of a component or components of a metered dose valve, which either come into contact with a movable component or are movable during storage or delivery from the medicinal inhalation device are treated according to methods described herein. Examples of such components for metered dose valves include e.g. seals, valve bodies, valve stems or compression springs of metered dose valves. More favorably said component may be selected from the group consisting of a valve body (e.g. a primary and/or a secondary valve body), a compression spring, a valve stem, a seal (e.g. inner and/or outer seal), and sub-assemblies comprising two or more of the aforesaid parts. Most favorably the component is a seal, a component that comes into sliding contact with the seal (e.g. valve stem or valve body, depending on the particular design of the metered dose valve) or a component comprising a seal (e.g. a core made of the valve stem, and one or more seals and if applicable a spring).

One type of singular component part or parts, e.g. seal or seals, may be treated or a mixture of singular component parts (e.g. valve stems and springs) made be treated together. Alternatively or additionally, as applicable, sub-assembly component(s) or in according to one aspect of present invention the whole valve itself may be treated.

In assembling a valve using a treated component to provide an assembled valve for attachment to the medicinal container (e.g. aerosol can of a pMDI) all the component parts making up the valve may be coated or more commonly only a certain part or certain parts of valve may be coated.

The step of forming a coating comprises contacting said at least a portion of a surface of the valve or the component, as applicable, with particulate material in dry form to provide said coating (dry coating); or contacting said at least a portion of a surface of the valve or the component, as applicable, with particulate material suspended in a fluid and then removing the fluid to provide particulate material coating (wet coating).

Dry coating may be conducted by mixing (e.g. shaking, tumbling, stirring) component(s) in the dry particulate material. It has been found that as increasing amounts of coating powder are applied, the surfaces facing outwardly, i.e. easy accessible surfaces, become so to say saturated with powder at a particular level of powder, leaving free powder to accumulate on the less accessible surfaces (e.g. the bore of a seal) of the component. Hence, when larger amounts of coating powder are applied, an increasingly larger proportion coats the less accessible surfaces until more or less a plateau of coating is reached. (Any excess particulate material may be removed by e.g. sifting.) Surprisingly it has been found that such less accessible surfaces, e.g. bores of seals, often receive a more thorough and uniform coating (i.e. a higher coating density) than easy accessible surfaces, e.g. faces and outer edges of seals, possibly due to a lack of or a minimal amount of "buffeting" of components against less accessible surfaces of other components during coating and/or, as applicable for example in the case of wet coating, an art turbophoresis effect.

Wet coating is conducted using a dispersion of the particulate material in the fluid . Fluids used in wet coating include a volatile hydrocarbon, a volatile alcohol, a volatile ester, a volatile fluorocarbon or a mixture thereof, more particularly n-hexane, n-heptane, ethanol, isopropanol, ethylacetate, HFE 7100 or a mixture thereof. Generally however it has been found desirable to use dispersions of particulate material for wet coating. In particular it has been found that concentrations of particulate material dispersions or solutions equal to or greater than 0.1 % w/v desirably facilitate film formation and films that provide a lubricating effect after removal of the fluid (e.g. drainage/evaporation of the fluid). Dispersions at concentrations greater than 25% w/v are generally impractical, the dispersion being quite viscous.

As indicated *supra,* for the provision of a coating having desirable lubricating properties, it is advantageous that the particulates of the particulate material are flat or plate-like.

For dry coating for desirable coat formation and/or subsequent coat properties, it has been found desirable that the particulates of the particulate materials are substantially completely de-agglomerated. For dry coating at least 90 % by weight of the particulate material passes or would pass, as applicable, a 325 mesh, in particular at least 90 % by weight of the particulate material passes or would pass, as applicable, a 400 mesh, more particularly at least 95 % by weight of that particulate material passes or would pass, a applicable, a 400 mesh.

For wet coating for desirable coat formation and/or subsequent coat properties, it has been found desirable that the particulates of the particulate materials are substantially completely de-agglomerated. For example, for wet coating such de-agglomeration of the suspended particulate material can be advantageously assured prior to contacting the component or components to the coating dispersion of particulate material, for example by high shear mixing the suspending fluid using a Silverson mixer while adding the particulate material. For wet coating, favorably at least 90 % by weight of the particulate material passes or would pass, as applicable, a 325 mesh, in particular at least 90 % by weight of the particulate material passes or would pass, as applicable, a 400 mesh, more particularly at least 95 % by weight of that particulate material passes or would pass, a applicable, a 400 mesh.

Additionally or alternatively, the mass median diameter of the particulates of the particulate material, e.g. as determined by Malvern Laser Diffraction, are favorably generally at most 30 microns, more favorably at most 25 micron, even more favorably even more favorably at most 20 micron and most favorably at most 15 micron. For wet coating, methods may advantageously include a process step or steps, such as high pressure homogenization, to produce a dispersion comprising submicron particulates (i.e. having a mass median diameter less than 1 micron).

Additionally or alternatively the specific surface area, e.g. as determined by a method based on the theory of Brunauer, Emmett and Teller, J. Am. Chem. Soc., vol. 60, p.577 (1960), e.g. using a Beckman Coulter SA 3100 Surface area and Pore size analyzer "BET specific surface area", of the particulate material is desirably equal to or greater than 2 m²/g, in particular greater than 3 m²/g.

Magnesium stearate and mixtures of magnesium stearate and magnesium palmitate are particularly preferred. Particulate material comprising crystalline magnesium stearate and/or palmitate are particularly preferred, in particular crystalline magnesium stearate and/or palmitate include an generally long lattice spacing, e.g. a d-spacing equal to or greater than 10, e.g. as determined by X-ray diffraction studies.

### Experimental Section

### Experiment 1 - Wet coating:

10g of Magnesium Stearate (NF ex FISCHER SCIENTIFIC Lot 432021, -10 micron, stearic acid ≥40%; total of stearic and palmitic acid > 90%) was added to dehydrated ethanol (400 g) and high shear mixed using a Silverson mixer for 1 minute. The dispersion was added to a product vessel of an Avestin C50 homogenizer and processed at 20,000 p.s.i. using recirculation for 30 minutes. Microscopic analysis of a sample taken from the resulting dispersion indicated that particulate material has a relatively uniform particle size of about 0.5 µm.

A plurality of valve cores (sub-assemblies consisting of stainless steel valve stem, a stainless steel valve spring, an outer diaphragm nitrile seal and an inner tank nitrile seal (see Figure 1a&b) were dipped in the prepared dispersion and then allowed to drain on a tray while letting the solvent evaporate.

Metered dose valves of the type shown in Figures 1 a & b were assembled from the coated cores and the other necessary, non coated component parts, i.e. metering tank, bottle emptier and, ferrule.

Inhalers were prepared by cold-filling formulation consisting of 1.97 mg/ml Albuterol Sulfate (having a majority of particles in the range of 1 to 3 microns) and HFA 134a into 10 ml aluminum cans, crimping the assembled valves to the cans and finally allowing the inhalers to warm to room temperature. The so-prepared inhalers were tested for force to fire, return force and friction as described below.

As a comparative, metered dose inhalers with siliconized (dimethicone) valves were prepared.

### Method of Force measurement

After allowing a period of 7 days for acclimatization of the seals in the aerosol units, the following is test method used for the force characteristics of the valve after actuating various numbers of doses through the life of the unit as prescribed below:
1. Insert the aerosol unit into a fresh actuator, and prime the inhaler, i.e. shake the inhaler with a gentle rocking action through 180° inversion for at least 10 seconds and immediately fire two shots to waste.
2. Weigh the aerosol unit with actuator, then fire one shot. Repeat five times, followed by a further weighing, and calculate five shot weights by subtracting weighings.
3. Fire two priming shots. Insert aerosol unit into a tensile tester and record the profile of force against valve travel for firing and return of the valve at 20 mm/min. Record the Force to fire the valve, corresponding to the point in the cycle where the stem side hole first breaks through the elastomeric outer seal seal during its inward travel. Record the Return Force for the valve, corresponding to the point in the cycle where the stem groove first breaks through the tank seal during its outward travel. Friction represents half the difference in outward and return forces at a prescribed valve stem travel.
4. Repeat step 3, two more times.
5. Fire 40 shots to waste using an automatic valve firing machine.
6. Repeat steps 1 to 5.
7. Repeat steps 1 to 4, then fire 40 shots to waste using an automatic valve firing machine.
8. Repeat steps 1 to 4.

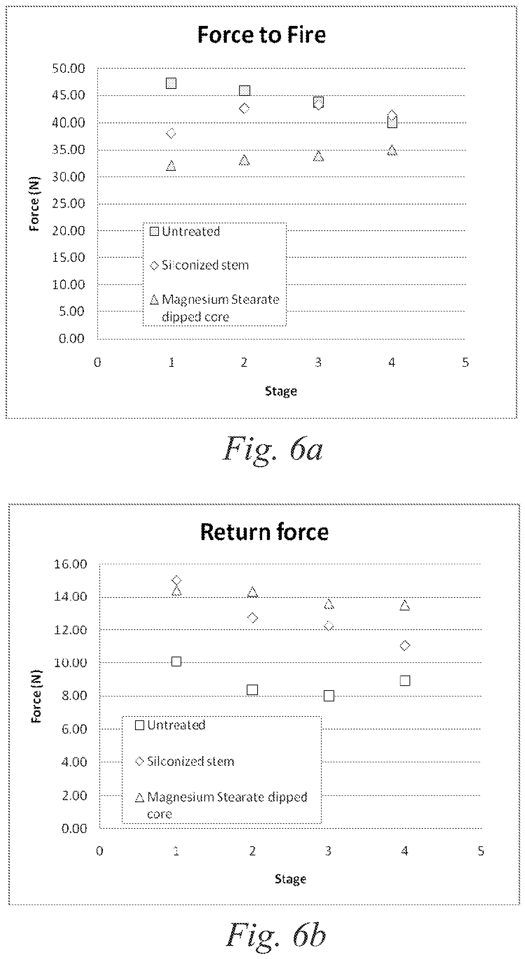

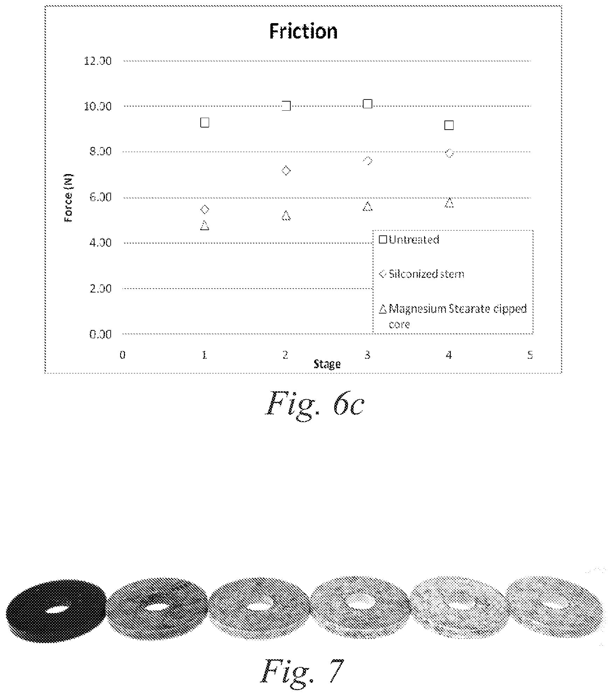

The four occasions that steps 1 to 4 are carried out are referred to as Stages 1 to 4 and represent shot umbers 22-30, 78-86, 134-142 and 190-198 respectively. The results are summarized in Figure 6 a to c; □ untreated; ◇ siliconized; and Δ magnesium stearate wet coated.

### Experiment 2 - Dry Coating:

Elastomer outer diaphragm nitrile seals (total weight 57 grams) were placed in a 250 ml beaker, and dry particulate Magnesium Stearate ((vegetable grade) PARTEC™ LUB MST ex MERCK Lot K41295363043; mass median diameter of particles about 5 microns.) in an amount as indicated in the following Table was added.

**Table 1**

| No. | Weight of powder (mg) | Whiteness of seal bore | Whiteness of seal edge | Whiteness of seal face |
|---|---|---|---|---|
| 2a | 65 | 83 ± 12 | 109 ± 15 | 104 ± 19 |
| 2b | 97 | 143 ± 14 | 158 ± 20 | 147 ± 17 |
| 2c | 130 | 176± 11 | 172 ± 21 | 158 ± 24 |
| 2d | 152 | 191 ± 8 | 184 ± 21 | 171 ± 20 |
| 2e | 165 | 184 ± 8 | 174 ± 23 | 156 ± 24 |
| | | | | |
| no coating | - | 28 ± 5 | 30 ± 2 | 19 ± 3 |

The beaker was closed with a film sold under the trademark PARAFILM, and the beaker was shaken for 30 seconds Thereafter, the coated seals were removed from the beaker. Coated seals and uncoated seals were photographed. The appearance of the elastomeric outer seal components is exemplified in the photograph shown in Figure 7, which shows, from left to right, uncoated, then coated with Magnesium Stearate at levels of 65, 97, 130, 152 and 165 mg respectively.

The level of whiteness given in Table 1 was measured using the following technique: The electronic photographs were opened in Corel Paint Shop Pro Photo X2 software using default settings. Ensure selection of Image>greyscale. Open the 'tools' toolbar and select the Dropper tool. In the Dropper palette, select 11x11 pixel size. Sample 10 colour readings from regions where the camera flash was directed in the bore of the coated components. The R, G and B readings will be equal for each reading, due to selection of the greyscale option. The readings represent "whiteness" on a scale of 0 to 255, and provide an indication of the extent of coating. The tabulated values show average whiteness together with the standard deviation of a set of 10 readings, each value representing the averaged results for 5 components.

Additional dry coated seals were prepared using Magnesium Stearate from FISCHER SCIENTIFIC (NF, Lot 432021) using the method described above with the amounts of Magnesium Stearate given in Table 2:

**Table 2**

| No. | Weight of powder (mg) |
|---|---|
| 2f | 65 |
| 2g | 130 |

### Angle of Slip

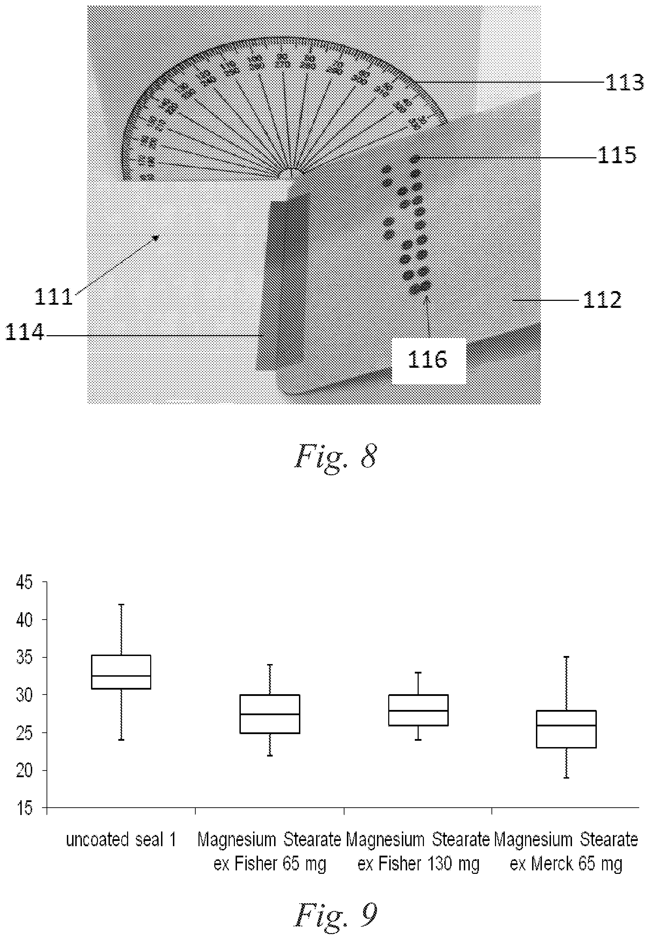

Uncoated seals and seals from Example No. 2a, 2f and 2g were measured for Angle of Slip using the apparatus (111) shown in Figure 8 and the following method: In the apparatus, a clipboard (112) was adhered at one edge to a flat horizontal surface by means of adhesive tape (114), to provide a hinge, and a protractor (113) is mounted vertically and orthogonally to the hinge line which intersects the central point on the protractor. Ten test seals (115) were affixed using double-sided adhesive tape in a line (116) at some distance from and parallel to the hinge. Thereafter one of the same batch of exemplary seals were mounted on top of each of the ten affixed seals (e.g. a 2a-exemplary seal was placed loose on a fixed 2a-exemplary seal). The clipboard was pivoted about the hinge by 1 degree increments with a 5 seconds dwell time at each new angle increment, and the angle at which each top seal slips off the seal underneath it was recorded. Figure 9 shows a box-plot of the results.

## Claims

1. A method of making a metered dose valve (10) for use in a medicinal inhalation device (100) or a component of a metered dose valve (10) for use in a medicinal inhalation device (100), wherein
at least a portion of a surface of the valve (10) or component, respectively, is to be coated, the method comprising the steps:
a) providing the valve (10) or the component, respectively; and
b) forming a coating on said at least a portion of a surface of the valve or the component, respectively, wherein said coating comprises a particulate material selected from the group consisting of Magnesium Stearate, Calcium Stearate, Zinc Stearate, Aluminium Stearate, Stearic acid, Magnesium Palmitate, Calcium Palmitate, Zinc Palmitate or Aluminium Palmitate, Palmitic acid, and mixtures thereof;
**characterized in that** the step of forming a coating on said at least a portion of a surface of the valve (10) or the component, respectively, comprises
contacting said at least a portion of a surface of the valve (10) or the component, as applicable, with particulate material in dry form to provide said coating, wherein the particulate material is de-agglomerated, such that at least 90 % by weight of the particulate material passes or would pass, as applicable, a 325 mesh; or
contacting said at least a portion of a surface of the valve (10) or the component, as applicable, with particulate material suspended in a fluid and then removing the fluid to provide said coating, wherein the fluid comprises a volatile hydrocarbon, a volatile alcohol, a volatile ester, a volatile fluorocarbon or a mixture thereof.

2. The method of making the metered dose valve of claim 1 for use in a medicinal inhalation device (100), wherein at least a portion of a surface of a component of the valve (10) is to be coated, the method comprising the steps:
a) providing the component of the valve (10);
b) forming the coating on said at least a portion of a surface of the component; and
c) assembling the valve (10) using said coated component and, as applicable, other valve-components.

3. A method according to claim 1 or claim 2, wherein the component is a singular part or a sub-assembly.

4. A method according to any one of claims 1 to 3, wherein the component is a component that comes in contact with a movable component or is movable during storage or delivery from the medicinal inhalation device (100).

5. A method according to any one of the preceding claims, wherein the component is a seal (16, 17) or the component comprises a seal (16, 17) or the component is a component which comes into contact with a seal (16, 17), in particular the component is a seal (16, 17) or a component comprising a seal (16, 17).

6. A method according to any one of the preceding claims, wherein the component is a seal (16, 17) and comprises Nitrile, EPDM, Neoprene, Butyl, Chlorobutyl or a thermoplastic elastomer.

7. A method according to any one of the preceding claims, wherein the component is a valve stem (14), in particular a valve stem (14) made of a material comprising Stainless Steel, Polyoxymethylene, Polybutylene terephthalate, Nylon, PEEK, polymethylpentene, polyphenylene sulphide, thermotropic liquid crystalline polymer or PTFE.

8. A method according to any one of the preceding claims, wherein the component is a spring (15), in particular a spring (15) made of Stainless steel.

9. A method according to any one of the preceding claims, wherein the component is a subassembly comprising a valve stem (14) and one or more seals (16, 17), in particular said subassembly further comprises a spring (15).

10. A method according to any one of the preceding claims, wherein the step of forming a coating on said at least a portion of a surface of the valve (10) or the component, respectively, comprises contacting said at least a portion of a surface of the valve (10) or the component, as applicable, with particulate material in dry form to provide said coating.

11. A method according to any one of the preceding claims, wherein the step of forming a coating on said at least a portion of a surface of the valve (10) or the component, respectively, comprises contacting said at least a portion of a surface of the valve (10) or the component, as applicable, with particulate material suspended in a fluid and then removing the fluid to provide said coating, wherein the fluid comprises n-hexane, n-heptane, ethanol, isopropanol, ethylacetate, HFE 7100 or a mixture thereof.

12. A method according to any one of the preceding claims, wherein the particulate material, suspended in a fluid, is de-agglomerated, such that at least 90 % by weight of the particulate material passes or would pass, as applicable, a 325 mesh; or wherein the particulate material is de-agglomerated, such that at least 90 % by weight of the particulate material passes or would pass, as applicable, a 400 mesh, in particularly at least 95 % by weight of that particulate material passes or would pass, as applicable, a 400 mesh; and/or
the mean median diameter of the particulates of the particulate material is at most 5 microns, in particular at most 25 microns, more particularly at most 20 microns, even more particularly at most 15 microns; and/or
the specific surface area of the particulate material is equal to or greater than 2 m²/g, in particular equal to or greater than 3 m²/g; and/or
the particulate material is flat or plate-like, in particular the mass mean aspect ratio of the particulates of the particulate material is equal to or greater than 5, more desirably equal to or greater than 10.

13. A method according to any one of the preceding claims, wherein the particulate material comprises Magnesium stearate, Magnesium palmitate or mixtures thereof.

14. A method according to claim 13, wherein the particulate material comprises crystalline Magnesium stearate, Magnesium palmitate or mixtures thereof, in particular crystalline Magnesium stearate, Magnesium palmitate or mixtures thereof having a d-spacing equal to or greater than 10.

15. A method according to any one of the preceding claims, wherein the medicinal inhalation device is a pressurized metered dose medicinal inhalation device, in particular a pressurized metered dose inhaler.

## Patentansprüche

1. Verfahren zur Herstellung eines Dosierventils (10) zur Verwendung in einer medizinischen Inhalationsvorrichtung (100) oder einer Komponente eines Dosierventils (10) zur Verwendung in einer medizinischen Inhalationsvorrichtung (100), wobei zumindest ein Teil einer Oberfläche des Ventils (10) bzw. der Komponente beschichtet werden soll, wobei das Verfahren die Schritte umfasst:
a) Bereitstellen des Ventils (10) bzw. der Komponente; und
b) Ausbilden einer Beschichtung auf dem zumindest einen Teil einer Oberfläche des Ventils bzw. der Komponente, wobei die Beschichtung ein teilchenförmiges Material, ausgewählt aus der Gruppe bestehend aus Magnesiumstearat, Calciumstearat, Zinkstearat, Aluminiumstearat, Stearinsäure, Magnesiumpalmitat, Calciumpalmitat, Zinkpalmitat oder Aluminiumpalmitat, Palmitinsäure, und Gemischen davon, umfasst;
**dadurch gekennzeichnet, dass** der Schritt des Ausbildens einer Beschichtung auf dem zumindest einen Teil einer Oberfläche des Ventils (10) bzw. der Komponente umfasst:
Inkontaktbringen des zumindest einen Teils einer Oberfläche des Ventils (10) oder der Komponente, wie zutreffend, mit teilchenförmigem Material in trockener Form, um die Beschichtung bereitzustellen, wobei das teilchenförmige Material so deagglomeriert wird, dass mindestens 90 Gew.-% des teilchenförmigen Materials, gegebenenfalls, ein 325 mesh passieren oder passieren würden; oder
Inkontaktbringen des zumindest einen Teils einer Oberfläche des Ventils (10) oder der Komponente, wie zutreffend, mit teilchenförmigem Material, suspendiert in einer Flüssigkeit, und danach Entfernen der Flüssigkeit, um die Beschichtung bereitzustellen, wobei die Flüssigkeit einen flüchtigen Kohlenwasserstoff, einen flüchtigen Alkohol, einen flüchtigen Ester, einen flüchtigen Fluorkohlenwasserstoff oder ein Gemisch davon umfasst.

2. Verfahren zur Herstellung des Dosierventils nach Anspruch 1 zur Verwendung in einer medizinischen Inhalationsvorrichtung (100), wobei zumindest ein Teil einer Oberfläche einer Komponente des Ventils (10) beschichtet werden soll, wobei das Verfahren die Schritte umfasst:
a) Bereitstellen der Komponente des Ventils (10);
b) Ausbilden der Beschichtung auf dem zumindest einen Teil einer Oberfläche der Komponente; und
c) Montieren des Ventils (10) unter Verwendung der beschichteten Komponente und, gegebenenfalls, weiterer Ventilkomponenten.

3. Verfahren nach Anspruch 1 oder Anspruch 2, wobei die Komponente ein Einzelteil oder eine Unterbaugruppe ist.

4. Verfahren nach einem der Ansprüche 1 bis 3, wobei die Komponente eine Komponente ist, die in Kontakt mit einer beweglichen Komponente kommt oder beweglich ist während der Lagerung oder Abgabe aus der medizinischen Inhalationsvorrichtung (100).

5. Verfahren nach einem der vorangehenden Ansprüche, wobei die Komponente eine Dichtung (16, 17) ist oder die Komponente eine Dichtung (16, 17) umfasst oder die Komponente eine Komponente, die in Kontakt mit einer Dichtung (16, 17) kommt, ist, insbesondere die Komponente eine Dichtung (16, 17) oder eine Komponente, umfassend eine Dichtung (16, 17), ist.

6. Verfahren nach einem der vorangehenden Ansprüche, wobei die Komponente eine Dichtung (16, 17) ist und Nitril, EPDM, Neopren, Butyl, Chlorbutyl oder ein thermoplastisches Elastomer umfasst.

7. Verfahren nach einem der vorangehenden Ansprüche, wobei die Komponente ein Ventilschaft (14), insbesondere ein Ventilschaft (14), hergestellt aus einem Material, umfassend nichtrostenden Stahl, Polyoxymethylen, Polybutylenterephthalat, Nylon, PEEK, Polymethylpenten, Polyphenylensulfid, thermotropes flüssigkristallines Polymer oder PTFE, ist.

8. Verfahren nach einem der vorangehenden Ansprüche, wobei die Komponente eine Feder (15), insbesondere eine Feder (15), hergestellt aus nichtrostendem Stahl, ist.

9. Verfahren nach einem der vorangehenden Ansprüche, wobei die Komponente eine Unterbaugruppe, umfassend einen Ventilschaft (14) und eine oder mehrere Dichtungen (16, 17), ist, insbesondere die Unterbaugruppe weiterhin eine Feder (15) umfasst.

10. Verfahren nach einem der vorangehenden Ansprüche, wobei der Schritt des Ausbildens einer Beschichtung auf dem zumindest einen Teil einer Oberfläche des Ventils (10) bzw. der Komponente das Inkontaktbringen des zumindest einen Teils einer Oberfläche des Ventils (10) oder der Komponente, wie zutreffend, mit teilchenförmigem Material in trockener Form, um die Beschichtung bereitzustellen, umfasst.

11. Verfahren nach einem der vorangehenden Ansprüche, wobei der Schritt des Ausbildens einer Beschichtung auf dem zumindest einen Teil einer Oberfläche des Ventils (10) bzw. der Komponente das Inkontaktbringen des zumindest einen Teils einer Oberfläche des Ventils (10) oder der Komponente, wie zutreffend, mit teilchenförmigem Material, suspendiert in einer Flüssigkeit, und danach das Entfernen der Flüssigkeit, um die Beschichtung bereitzustellen, umfasst, wobei die Flüssigkeit n-Hexan, n-Heptan, Ethanol, Isopropanol, Ethylacetat, HFE 7100 oder ein Gemisch davon umfasst.

12. Verfahren nach einem der vorangehenden Ansprüche, wobei das teilchenförmige Material, suspendiert in einer Flüssigkeit, so deagglomeriert wird, dass mindestens 90 Gew.-% des teilchenförmigen Materials, gegebenenfalls, ein 325 mesh passieren oder passieren würden; oder wobei das teilchenförmige Material, suspendiert in einer Flüssigkeit, so deagglomeriert wird, dass mindestens 90 Gew.-% des teilchenförmigen Materials, gegebenenfalls, ein 400 mesh passieren oder passieren würden, insbesondere mindestens 95 Gew.-% dieses teilchenförmigen Materials, gegebenenfalls, ein 400 mesh passieren oder passieren würden; und/oder der mittlere Mediandurchmesser der Teilchen des teilchenförmigen Materials höchstens 5 µm, insbesondere höchstens 25 µm, weiter insbesondere höchstens 20 µm, noch weiter insbesondere höchstens 15 µm beträgt; und/oder
die spezifische Oberfläche des teilchenförmigen Materials gleich oder größer als 2 m²/g, insbesondere gleich oder größer als 3 m²/g ist; und/oder
das teilchenförmige Material flach oder plättchenförmig ist, insbesondere das massenmittlere Aspektverhältnis der Teilchen des teilchenförmigen Materials gleich oder größer als 5, stärker erwünscht gleich oder größer als 10 ist.

13. Verfahren nach einem der vorangehenden Ansprüche, wobei das teilchenförmige Material Magnesiumstearat, Magnesiumpalmitat oder Gemische davon umfasst.

14. Verfahren nach Anspruch 13, wobei das teilchenförmige Material kristallines Magnesiumstearat, Magnesiumpalmitat oder Gemische davon, insbesondere kristallines Magnesiumstearat, Magnesiumpalmitat oder Gemische davon mit einem d-Abstand gleich oder größer als 10, umfasst.

15. Verfahren nach einem der vorangehenden Ansprüche, wobei die medizinische Inhalationsvorrichtung eine unter Druck stehende medizinische Dosierinhalationsvorrichtung, insbesondere ein unter Druck stehender Dosierinhalator, ist.

## Revendications

1. Procédé de fabrication d'une vanne à dose mesurée (10) destinée à être utilisée dans un dispositif d'inhalation médical (100) ou d'un composant d'une vanne à dose mesurée (10) destiné à être utilisé dans un dispositif d'inhalation médical (100), dans lequel
au moins une partie d'une surface de la vanne (10) ou du composant, respectivement, doit être revêtue, le procédé comprenant les étapes consistant à :
a) fournir la vanne (10) ou le composant, respectivement ; et
b) former un revêtement sur ladite au moins une partie d'une surface de la vanne ou du composant, respectivement, ledit revêtement comprenant un matériau particulaire choisi dans le groupe constitué par le stéarate de magnésium, le stéarate de calcium, le stéarate de zinc, le stéarate d'aluminium, l'acide stéarique, le palmitate de magnésium, le palmitate de calcium, le palmitate de zinc ou le palmitate d'aluminium, l'acide palmitique et leurs mélanges ;
**caractérisé en ce que** l'étape de formation d'un revêtement sur ladite au moins une partie d'une surface de la vanne (10) ou du composant, respectivement, comprend
la mise en contact de ladite au moins une partie d'une surface de la vanne (10) ou du composant, comme applicable, avec le matériau particulaire sous forme anhydre pour fournir ledit revêtement, le matériau particulaire étant désaggloméré, de telle manière qu'au moins 90 % en poids du matériau particulaire passe ou passerait, comme applicable, un mesh de 325 ; ou
la mise en contact de ladite au moins une partie d'une surface de la vanne (10) ou du composant, comme applicable, avec le matériau particulaire en suspension dans un fluide puis l'enlèvement du fluide pour fournir ledit revêtement, le fluide comprenant un hydrocarbure volatil, un alcool volatil, un ester volatil, un fluorocarbone volatil ou un de leurs mélanges.

2. Procédé de fabrication de la vanne à dose mesurée selon la revendication 1 destinée à être utilisée dans un dispositif d'inhalation médical (100), dans lequel au moins une partie d'une surface d'un composant de la vanne (10) doit être revêtue, le procédé comprenant les étapes consistant à :
a) fournir le composant de la vanne (10) ;
b) former le revêtement sur ladite au moins une partie d'une surface du composant ; et
c) assembler la vanne (10) en utilisant ledit composant revêtu et, comme applicable, d'autres composants de vanne.

3. Procédé selon la revendication 1 ou la revendication 2, dans lequel le composant est une partie singulière ou un sous-assemblage.

4. Procédé selon l'une quelconque des revendications 1 à 3, dans lequel le composant est un composant qui vient en contact avec un composant amovible ou est amovible durant le stockage ou la délivrance du dispositif d'inhalation médical (100) .

5. Procédé selon l'une quelconque des revendications précédentes, dans lequel le composant est un joint (16, 17) ou le composant comprend un joint (16 ; 17) ou le composant est un composant qui vient en contact avec un joint (16, 17), en particulier le composant est un joint (16, 17) ou un composant comprenant un joint (16, 17).

6. Procédé selon l'une quelconque des revendications précédentes, dans lequel le composant est un joint (16, 17) et comprend du nitrile, de l'EPDM, du néoprène, du butyle, du chlorobutyle ou un élastomère thermoplastique.

7. Procédé selon l'une quelconque des revendications précédentes, dans lequel le composant est une tige de vanne (14), en particulier une tige de vanne (14) constituée d'un matériau comprenant de l'acier inoxydable, du polyoxyméthylène, du téréphtalate de polybutylène, du nylon, du PEEK, du polyméthylpentène, du sulfure de polyphénylène, un polymère cristallin liquide thermotropique ou du PTFE.

8. Procédé selon l'une quelconque des revendications précédentes, dans lequel le composant est un ressort (15), en particulier un ressort (15) constitué d'acier inoxydable.

9. Procédé selon l'une quelconque des revendications précédentes, dans lequel le composant est un sous-assemblage comprenant une tige de vanne (14) et un ou plusieurs joints (16, 17), en particulier ledit sous-assemblage comprend en outre un ressort (15).

10. Procédé selon l'une quelconque des revendications précédentes, dans lequel l'étape de formation d'un revêtement sur ladite au moins une partie d'une surface de la vanne (10) ou du composant, respectivement, comprend la mise en contact de ladite au moins une partie d'une surface de la vanne (10) ou du composant, comme applicable, avec un matériau particulaire sous forme anhydre pour fournir ledit revêtement.

11. Procédé selon l'une quelconque des revendications précédentes, dans lequel l'étape de formation d'un revêtement sur ladite au moins une partie d'une surface de la vanne (10) ou du composant, respectivement, comprend la mise en contact de ladite au moins une partie d'une surface de la vanne (10) ou du composant, comme applicable, avec un matériau particulaire en suspension dans un fluide puis l'enlèvement du fluide pour fournir ledit revêtement, le fluide comprenant le n-hexane, le n-heptane, l'éthanol, l'isopropanol, l'éthylacétate, le HFE 7100 ou un de leurs mélanges.

12. Procédé selon l'une quelconque des revendications précédentes, dans lequel le matériau particulaire, en suspension dans un fluide, est désaggloméré de telle manière qu'au moins 90 % en poids du matériau particulaire passe ou passerait, comme applicable, un mesh de 325 ; ou le matériau particulaire étant désaggloméré, de telle manière qu'au moins 90 % en poids du matériau particulaire passe ou passerait, comme applicable, un mesh de 400, en particulier au moins 95 % en poids de ce matériau particulaire passe ou passerait, comme applicable, un mesh de 400 ; et/ou
le diamètre médian moyen des particules du matériau particulaire est d'au plus 5 micromètres, en particulier d'au plus 25 micromètres, plus particulièrement d'au plus 20 micromètres, encore plus particulièrement d'au plus 15 micromètres ; et/ou
la surface spécifique du matériau particulaire est supérieure ou égale à 2 m²/g, en particulier supérieure ou égale à 3 m²/g ; et/ou
le matériau particulaire est plat ou de type plat, en particulier le rapport d'aspect moyen en masse des particules du matériau particulaire est supérieur ou égal à 5, de manière plus souhaitable supérieur ou égal à 10.

13. Procédé selon l'une quelconque des revendications précédentes, dans lequel le matériau particulaire comprend du stéarate de magnésium, du palmitate de magnésium ou leurs mélanges.

14. Procédé selon la revendication 13, dans lequel le matériau particulaire comprend du stéarate de magnésium cristallin, du palmitate de magnésium ou leurs mélanges, en particulier du stéarate de magnésium cristallin, du palmitate de magnésium ou leurs mélanges ayant un espacement d supérieur ou égal à 10.

15. Procédé selon l'une quelconque des revendications précédentes, dans lequel le dispositif d'inhalation médical est un dispositif d'inhalation médical à dose mesurée pressurisée, en particulier un inhalateur à dose mesurée pressurisée.
